(19) Europäisches Patentamt
European Patent Office
Office européen des brevets

(11) EP 2 796 562 A1

(12) EUROPEAN PATENT APPLICATION
published in accordance with Art. 153(4) EPC

(43) Date of publication:
**29.10.2014 Bulletin 2014/44**

(21) Application number: **12859103.9**

(22) Date of filing: **06.12.2012**

(51) Int Cl.:
***C12P 41/00*** *(2006.01)* ***C12P 13/04*** *(2006.01)*
***C12N 9/18*** *(2006.01)*

(86) International application number:
**PCT/JP2012/082274**

(87) International publication number:
**WO 2013/094499 (27.06.2013 Gazette 2013/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2011 JP 2011277194**

(71) Applicant: **Sumitomo Chemical Co., Ltd**
**Chuo-ku, Tokyo 104-8260 (JP)**

(72) Inventors:
- **AKIYAMA, Toshihiko**
**Osaka-shi**
**Osaka 555-0021 (JP)**
- **HIRATA, Norihiko**
**Osaka-shi**
**Osaka 555-0021 (JP)**

(74) Representative: **Nieuwenhuys, William Francis**
**Marks & Clerk LLP**
**90 Long Acre**
**London**
**WC2E 9RA (GB)**

# (54) METHOD FOR PRODUCING OPTICALLY-ACTIVE -SUBSTITUTED- -AMINO ACID

(57) The invention provides a method for producing an optically active α,-substituted-β-amino acid represented by formula (2), the method comprising a step in which an optical isomer mixture of an α-substituted-β-amino acid ester represented by formula (1) is subjected to asymmetric hydrolysis by using an enzyme and such capable of asymmetrically hydrolyzing the ester site of said optical isomer mixture.

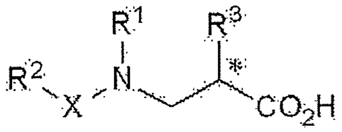

(2)

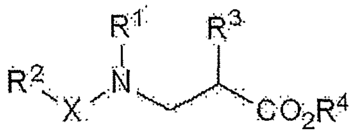

(1)

(In the formulae, $R^1$ represents an acyl group, a carboxyl group, or a carboxamide group; $R^2$ represents an alkyl group, an aryl group, an aralkyl group, a cyclic ether group, or the like; $R^3$ represents an optionally-substituted $C_1$ to $C_{20}$ hydrocarbon group; $R^4$ represents an alkyl group; X represents an oxygen atom or a sulfur atom; and * represents the asymmetric center.)

EP 2 796 562 A1

SEQUENCE LISTING

<110>  Sumitomo Chemical Co.,Ltd.

<120>  Method for producing optical active a-substituted-b-amino acid

<130>  PN814193EP

<140>  PCT/JP2012/082274
<141>  2012-12-06

<150>  JP 2011-277194
<151>  2011-12-19

<160>  1

<210>  1
<211>  370
<212>  PRT
<213>  Chromobacterium

<400>  1

```
Met Thr Leu Phe Asp Gly Ile Thr Ser Arg Ile Val Asp Thr Asp Arg
1               5                   10                  15

Leu Thr Val Asn Ile Leu Glu Arg Ala Ala Asp Asp Pro Gln Thr Pro
            20                  25                  30

Pro Asp Arg Thr Val Val Phe Val His Gly Asn Val Ser Ser Ala Leu
        35                  40                  45

Phe Trp Gln Glu Ile Met Gln Asp Leu Pro Ser Asp Leu Arg Ala Ile
    50                  55                  60

Ala Val Asp Leu Arg Gly Phe Gly Gly Ser Glu His Ala Pro Val Asp
65                  70                  75                  80

Ala Thr Arg Gly Val Arg Asp Phe Ser Asp Asp Leu His Ala Thr Leu
                85                  90                  95

Glu Ala Leu Asp Ile Pro Val Ala His Leu Val Gly Trp Ser Met Gly
            100                 105                 110

Gly Gly Val Val Met Gln Tyr Ala Leu Asp His Pro Val Leu Ser Leu
        115                 120                 125

Thr Leu Gln Ser Pro Val Ser Pro Tyr Gly Phe Gly Gly Thr Arg Arg
    130                 135                 140

Asp Gly Ser Arg Leu Thr Asp Asp Asp Ala Gly Cys Gly Gly Gly Gly
145                 150                 155                 160
```

```
Ala Asn Pro Asp Phe Ile Gln Arg Leu Ile Asp His Asp Thr Ser Asp
                165                 170                 175

Asp Ala Gln Thr Ser Pro Arg Ser Val Phe Arg Ala Gly Tyr Val Ala
            180                 185                 190

Ser Asp Tyr Thr Thr Asp His Glu Asp Val Trp Val Glu Ser Met Leu
        195                 200                 205

Thr Thr Ser Thr Ala Asp Gly Asn Tyr Pro Gly Asp Ala Val Pro Ser
    210                 215                 220

Asp Asn Trp Pro Gly Phe Ala Ala Gly Arg His Gly Val Leu Asn Thr
225                 230                 235                 240

Met Ala Pro Gln Tyr Phe Asp Val Ser Gly Ile Val Asp Leu Ala Glu
                245                 250                 255

Lys Pro Pro Ile Leu Trp Ile His Gly Thr Ala Asp Ala Ile Val Ser
            260                 265                 270

Asp Ala Ser Phe Tyr Asp Leu Asn Tyr Leu Gly Gln Leu Gly Ile Val
        275                 280                 285

Pro Gly Trp Pro Gly Glu Asp Val Ala Pro Ala Gln Glu Met Val Ser
    290                 295                 300

Gln Thr Arg Asp Val Leu Gly Arg Tyr Ala Ala Gly Gly Gly Thr Val
305                 310                 315                 320

Thr Glu Val Ala Val Glu Gly Ala Gly His Ser Ala His Leu Glu Arg
                325                 330                 335

Pro Ala Val Phe Arg His Ala Leu Leu Glu Ile Ile Gly Tyr Val Gly
            340                 345                 350

Ala Ala Ala Asp Pro Ala Pro Pro Thr Glu Ala Ile Ile Ile Arg Ser
        355                 360                 365

Ala Asp
    370
```

## Description

### TECHNICAL FIELD

The present invention relates to a method for producing an optically-active $\alpha$-substituted-$\beta$-amino acid.

### BACKGROUND ART

An optically-active $\alpha$-substituted-$\beta$-amino acid represented by formula (2):

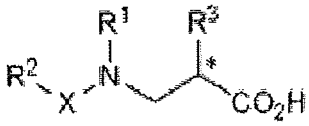
(2)

(wherein $R^1$ represents a hydrogen atom, a $C_1$ to $C_{10}$ acyl group, a carboxyl group or a carboxamide group; $R^2$ represents a hydrogen atom, an optionally-substituted $C_1$ to $C_{10}$ alkyl group, an optionally-substituted $C_6$ to $C_{20}$ aryl group, an optionally-substituted $C_7$ to $C_{20}$ aralkyl group, or a $C_2$ to $C_{20}$ acyl group, an optionally-substituted $C_2$ to $C_{15}$ alkoxycarbonyl group, an optionally-substituted $C_7$ to $C_{20}$ aryloxycarbonyl group, an optionally-substituted $C_8$ to $C_{20}$ aralkyloxycarbonyl group, or an optionally-substituted $C_4$ to $C_{10}$ cyclic ether group; $R^3$ represents an optionally-substituted $C_1$ to $C_{20}$ hydrocarbon group; X represents an oxygen atom or a sulfur atom; and * represents the asymmetric center.)
is useful as a raw material or an intermediate compound for producing an active pharmaceutical ingredient compound.
WO 00/61134, for example, describes a method for producing an optically-active $\alpha$-cyclopentylmethyl-$\beta$-(N-formyl-N-hydroxy) amino acid that is a raw material for producing an antimicrobial.

### SUMMARY OF THE INVENTION

The present invention provides a novel method for producing the optically-active $\alpha$-substituted-$\beta$-amino acid represented by formula (2).
That is, the present invention includes the following inventions [1] to [12]:

[1] A method for producing an optically-active $\alpha$-substituted-$\beta$-amino acid represented by formula (2) (hereinafter, referred to as "optically-active $\alpha$-substituted-$\beta$-amino acid (2)" in some cases), the method comprising a step of asymmetrically hydrolyzing an optical isomer mixture of an $\alpha$-substituted-$\beta$-amino acid ester represented by formula (1):

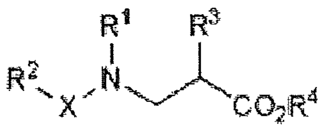
(1)

(wherein $R^4$ represents an optionally-substituted $C_1$ to $C_{10}$ alkyl group; and $R^1$, $R^2$, $R^3$ and X have the same meanings as described above.)
(hereinafter, referred to as "$\alpha$-substituted-$\beta$-amino acid ester (1)" in some cases)
using an enzyme capable of asymmetrically hydrolyzing the $-CO_2R^4$ group (hereinafter, referred to as "ester site" in some cases) of the optical isomer mixture or a culture of a microorganism capable of producing the enzyme or a treated product thereof.
[2] The method according to [1], wherein $R^3$ is an optionally-substituted $C_1$ to $C_{10}$ hydrocarbon group.
[3] The method according to [1], wherein $R^3$ is a cyclopentylmethyl group.
[4] The method according to any one of [1] to [3], wherein $R^1$ is a $C_1$ to $C_{10}$ acyl group.
[5] The method according to any one of [1] to [3], wherein $R^1$ is a formyl group, an acetyl group, a propionyl group or a benzoyl group.
[6] The method according to any one of [1] to [3], wherein $R^1$ is a hydrogen group.
[7] The method according to any one of [1] to [6], wherein $R^2$ is a $C_7$ to $C_{20}$ aralkyl group.
[8] The method according to any one of [1] to [6], wherein $R^2$ is a benzyl group.
[9] The method according to any one of [1] to [8], wherein $R^4$ is a methyl group or an ethyl group.

[10] The method according to any one of [1] to [9], wherein the enzyme is a hydrolase originated from *Chromobacterium spp.* or *Mucor spp.* microorganism or a variant-type enzyme obtained by deletion, addition or substitution of 1 or 2 of amino acids constituting the hydrolase.

[11] The method according to any one of [1] to [9], wherein the enzyme is a hydrolase originated from *Chromobacterium chocolatum* or *Mucor miehei* microorganism or a variant-type enzyme obtained by deletion, addition or substitution of 1 or 2 of amino acids constituting the hydrolase.

[12] The method according to any one of [1] to [9], wherein the enzyme is an esterase or lipase derived from *Chromobacterium* SC-YM-1 strain (Agency of Industrial Science and Technology: National Institute of Bioscience and Human Technology, accession number: FERM BP-6703) or a variant-type enzyme obtained by deletion, addition or substitution of 1 or 2 of amino acids constituting the esterase or lipase.

MODE FOR CARRYING OUT THE INVENTION

Hereinbelow, the production method of the present invention will be described in detail.

First, the present production method uses an optical isomer mixture of the α-substituted-β-amino acid ester (1).

$R^1$ in formula (1) represents a hydrogen atom, a $C_1$ to $C_{10}$ acyl group, a carboxyl group (-COOH) or a carboxamide group (-$CONH_2$). Examples of the acyl group include a formyl group, an acetyl group, a propionyl group, a butyryl group, a valeryl group, a pivaloyl group, and a benzoyl group, and these may be any of linear, branched and cyclic. Among them, a hydrogen atom, a formyl group, an acetyl group, a propionyl group and a benzoyl group are preferred, and a formyl group, an acetyl group and a propionyl group are particularly preferred. In addition, these acyl groups may be a substituted alkylcarbonyl group or a substituted arylcarbonyl group, and the substituent may be a $C_1$ to $C_5$ alkyl group, a $C_1$ to $C_5$ alkoxy group, a $C_7$ to $C_9$ aralkyloxy group, a halogen atom, a cyano group, a nitro group, or the like. Examples of the substituted acyl group include a chloroacetyl group, a dichloroacetyl group, a methoxyacetyl group, a 3-chloropropionyl group, a toluoyl group, a trifluoromethylbenzoyl group, a chlorobenzoyl group, a fluorobenzoyl group, a methoxybenzoyl group, a cyanobenzoyl group, and a nitrobenzoyl group. A raw material for producing an active pharmaceutical ingredient compound like the optically-active α-cyclopentylmethyl-β-(N-formyl-N-hydroxy)amino acid described in WO 00/61134 or an intermediate compound can be easily obtained from an α-substituted-β-amino acid ester derivative wherein $R^1$ is a hydrogen atom or a formyl group.

$R^2$ in formula (1) represents a hydrogen atom, an optionally-substituted $C_1$ to $C_{10}$ alkyl group, an optionally-substituted $C_6$ to $C_{20}$ aryl group, an optionally-substituted $C_7$ to $C_{20}$ aralkyl group, a $C_2$ to $C_{20}$ acyl group, an optionally-substituted $C_2$ to $C_{15}$ alkoxycarbonyl group, an optionally-substituted $C_7$ to $C_{20}$ aryloxycarbonyl group, an optionally-substituted $C_8$ to $C_{20}$ aralkyloxycarbonyl group, or an optionally-substituted $C_4$ to $C_{10}$ cyclic ether group.

Examples of the alkyl group include a methyl group, an ethyl group, a propyl group, a butyl group, a hexyl group, an octyl group, a decyl group and the like, and these groups may be any of linear, branched and cyclic. Among the alkyl group, a methyl group and a tert-butyl group are preferred.

Examples of the aryl group include a phenyl group, a naphthyl group, an anthracenyl group, and a biphenyl group, and among them, a phenyl group is preferred.

Examples of the aralkyl group include a benzyl group, a p-methoxybenzyl group, and a naphthylmethyl group, and among them, a benzyl group is preferred.

Examples of the acyl group include an acetyl group, a propionyl group, a butyryl group, a valeryl group, a pivaloyl group, and a benzoyl group, and among them, an acetyl group and a pivaloyl group are preferred.

Examples of the alkoxycarbonyl group include a methoxycarbonyl group, an ethoxycarbonyl group, a propoxycarbonyl group, a butoxycarbonyl group, a hexyloxycarbonyl group, an octyloxycarbonyl group, and a decyloxycarbonyl group, and these groups may be any of linear, branched and cyclic. Among them, a tert-butoxycarbonyl group is preferred.

Examples of the aryloxycarbonyl group include a phenoxycarbonyl group, a naphthyloxycarbonyl group, an anthracenyloxycarbonyl group, and a biphenyloxycarbonyl group, and among them, a phenoxycarbonyl group is preferred.

Examples of the aralkyloxycarbonyl group include a benzyloxycarbonyl group, and a naphthylmethoxycarbonyl group, and among them, a benzyloxycarbonyl group is preferred.

The cyclic ether group is a group obtained by removing one hydrogen atom from the cyclic ether compound, and the hydrogen atom removed from the cyclic ether compound is particularly preferably a hydrogen atom at the position 2 of the cyclic ether compound. Specific examples of the cyclic ether group include a tetrahydro-2H-pyran-2-yl group, a tetrahydrofuran-2-yl group and a 1,4-dioxan-2-yl group, and among them, a tetrahydro-2H-pyran-2-yl group is preferred.

In addition, these alkyl groups, aryl groups, aralkyl groups, alkoxycarbonyl groups, aryloxycarbonyl groups, aralkyloxycarbonyl groups and cyclic ether groups are optionally substituted, and the substituent can be a $C_1$ to $C_5$ alkoxy group, a $C_7$ to $C_{11}$ aralkyloxy group, a halogen atom, and the like. In addition, the group having an aromatic ring such as an aryl group, an aralkyl group, an aryloxycarbonyl group or an aralkyloxycarbonyl group may have a $C_1$ to $C_5$ alkyl group, a $C_1$ to $C_5$ alkoxy group or a halogen atom in its aromatic ring as a substituent.

$R^2$ is described with reference to specific examples thereof as above, and among them, $R^2$ is preferably a $C_7$ to $C_{20}$ aralkyl group, still more preferably a benzyl group or a p-methoxybenzyl group, and particularly preferably a benzyl group.

$R^3$ in formula (1) is a $C_1$ to $C_{20}$ hydrocarbon group, and the hydrocarbon group can be any of an aliphatic hydrocarbon group, an alicyclic hydrocarbon group or an aromatic hydrocarbon group, or a combination thereof. The carbon number of the hydrocarbon group of $R^3$ is preferably 1 to 7, and more preferably 3 to 6. The aliphatic hydrocarbon group is typically an alkyl group, and examples thereof include a methyl group, an ethyl group, a propyl group, a butyl group, a pentyl group, a hexyl group, an octyl group, a decyl group, a dodecyl group, a tetradecyl group, a hexadecyl group, an octadecyl group, and an icosyl group, and these groups may be linear or branched. The alicyclic hydrocarbon group includes a cyclobutyl group, a cyclopentyl group, a cyclohexyl group, a cyclooctyl group, a cyclodecyl group, a norbornyl group, an adamantyl group, and the like. The aromatic hydrocarbon group is typically an aryl group, and specific examples of the aryl group are the same as those exemplified as the aryl group of $R^2$. In addition, the hydrocarbon group of $R^3$ may also be optionally substituted, and the substituent is the same as those exemplified as the substituents of the alkyl group and aryl group of $R^2$. The carbon number of the optionally-substituted hydrocarbon group is preferably 1 to 10, and more preferably 3 to 7.

$R^3$ in formula (1) can be, as described above, a combination of an aliphatic hydrocarbon group and an alicyclic hydrocarbon group, a combination of an aliphatic hydrocarbon group and an aromatic hydrocarbon group, or a combination of an alicyclic hydrocarbon group and an aromatic hydrocarbon group. The combination of an aliphatic hydrocarbon group and an alicyclic hydrocarbon group is typically a combination of a cycloalkyl group and an alkanediyl group, and the like. It is specifically a cyclopentylmethyl group, a cyclopentylethyl group, a cyclopentylpropyl group, a cyclopentylbutyl group, a cyclohexylmethyl group, a cyclohexylethyl group, a cyclohexylpropyl group, a cyclohexylbutyl group, a cyclooctylmethyl group, a cyclooctylethyl group, a cyclooctylpropyl group, a cyclooctylbutyl group, and the like. The combination of an aliphatic hydrocarbon group and an aromatic hydrocarbon group is typically an aralkyl group, for example, a benzyl group, and a naphthylmethyl group. The combination of an alicyclic hydrocarbon group and an aromatic hydrocarbon group can be a phenylcyclopentyl group, a phenylcyclohexyl group, a naphthylcyclopentyl group, a naphthylcyclohexyl group, and the like.

$R^3$ is described with reference to specific examples thereof as above, and among them, $R^3$ is preferably a combination of an aliphatic hydrocarbon group and an alicyclic hydrocarbon group, further preferably a cyclopentylmethyl group or a cyclohexylmethyl group, and particularly preferably a cyclopentylmethyl group.

$R^4$ in formula (1) is an optionally-substituted $C_1$ to $C_{10}$ alkyl group, and the alkyl group maybe linear or branched. In addition, specific examples of the alkyl group are the same as those described in the alkyl group of $R^3$ with a carbon number in the range of 1 to 10. The alkyl group of $R^4$ has further preferably a carbon number of 1 to 4, and is particularly preferably a methyl group or ethyl group. Incidentally, the optional substituent of $R^4$ is the same as those exemplified as the optional substituent of $R^3$.

X in formula (1) represents an oxygen atom or a sulfur atom, and is preferably an oxygen atom.

Herein, specific examples of the preferred α-substituted-β-amino acid ester (1) are shown in Table 1 by combinations of $R^1$ to $R^4$ in formula (1).

[Table 1]

| α-substituted-β-amino acid | $R^1$ | $R^2$ | $R^3$ | $R^4$ |
|---|---|---|---|---|
| (1-1) | -H | Benzyl | 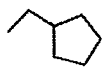 | $-CH_3$ |
| (1-2) | -H | Benzyl | 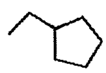 | $-C_2H_5$ |
| (1-3) | -H | Benzyl | 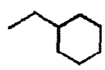 | $-CH_3$ |
| (1-4) | -H | Benzyl | 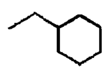 | $-C_2H_5$ |
| (1-5) | -H | 4-Methoxybenzyl | 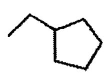 | $-CH_3$ |
| (1-6) | -H | 4-Methoxybenzyl | 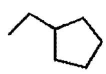 | $-C_2H_5$ |

(continued)

| α-substituted-β-amino acid | $R^1$ | $R^2$ | $R^3$ | $R^4$ |
|---|---|---|---|---|
| (1-7) | -H | 4-Methoxybenzyl | 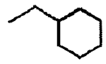 | $-CH_3$ |
| (1-8) | -H | 4-Methoxybenzyl | 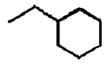 | $-C_2H_5$ |
| (1-9) | -CHO | Benzyl | 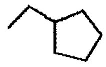 | $-CH_3$ |
| (1-10) | -CHO | Benzyl | 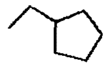 | $-C_2H_5$ |
| (1-11) | -CHO | Benzyl | 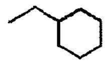 | $-CH_3$ |
| (1-12) | -CHO | Benzyl | 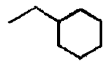 | $-C_2H_5$ |
| (1-13) | -CHO | 4-Methoxybenzyl | 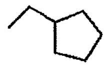 | $-CH_3$ |
| (1-14) | -CHO | 4-Methoxybenzyl | 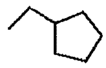 | $-C_2H_5$ |
| (1-15) | -CHO | 4-Methoxybenzyl | 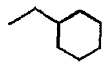 | $-CH_3$ |
| (1-16) | -CHO | 4-Methoxybenzyl | 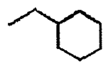 | $-C_2H_5$ |
| (1-17) | $-C(O)CH_3$ | Benzyl | 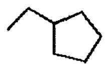 | $-CH_3$ |
| (1-18) | $-C(O)CH_3$ | Benzyl | 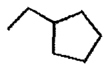 | $-C_2H_5$ |
| (1-19) | $-C(O)CH_3$ | Benzyl | 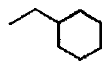 | $-CH_3$ |
| (1-20) | $-C(O)CH_3$ | Benzyl | 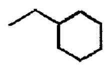 | $-C_2H_5$ |
| (1-21) | $-C((O))CH_3$ | 4-Methoxybenzyl | 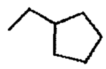 | $- CH_3$ |
| (1-22) | $-C(O)CH_3$ | 4-Methoxybenzyl | 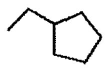 | $-C_2H_5$ |
| (1-23) | $-C(O)CH_3$ | 4-Methoxybenzyl | 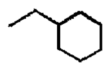 | $-CH_3$ |
| (1-24) | $-C(O)CH_3$ | 4-Methoxybenzyl | 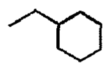 | $-C_2H_5$ |
| (1-25) | $-C(O)C_2H_5$ | Benzyl | 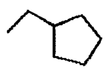 | $-CH_3$ |

(continued)

| α-substituted-β-amino acid | $R^1$ | $R^2$ | $R^3$ | $R^4$ |
|---|---|---|---|---|
| (1-26) | $-C(O)C_2H_5$ | Benzyl | 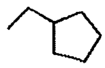 | $-C_2H_5$ |
| (1-27) | $-C(O)C_2H_5$ | Benzyl | 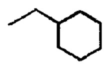 | $-CH_3$ |
| (1-28) | $-C(O)C_2H_5$ | Benzyl | 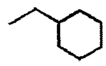 | $-C_2H_5$ |
| (1-29) | $-C(O)C_2H_5$ | 4-Methoxybenzyl | 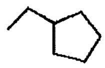 | $-CH_3$ |
| (1-30) | $-C(O)C_2H_5$ | 4-Methoxybenzyl | 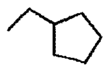 | $-C_2H_5$ |
| (1-31) | $-C(O)C_2H_5$ | 4-Methoxybenzyl | 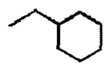 | $-CH_3$ |
| (1-32) | $-COC_2H_5$ | 4-Methoxybenzyl | 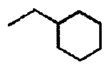 | $-C_2H_5$ |
| (1-33) | $-C(O)C_6H_5$ | Benzyl | 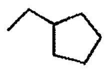 | $-CH_3$ |
| (1-34) | $-C(O)C_6H_5$ | Benzyl | 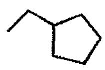 | $-C_2H_5$ |
| (1-35) | $-C(O)C_6H_5$ | Benzyl | 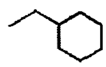 | $- CH_3$ |
| (1-36) | $-C(O)C_6H_5$ | Benzyl | 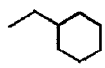 | $-C_2H_5$ |
| (1-37) | $-C(O)C_6H_5$ | 4-Methoxybenzyl | 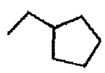 | $-CH_3$ |
| (1-38) | $-C(O)C_6H_5$ | 4-Methoxybenzyl | 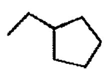 | $-C_2H_5$ |
| (1-39) | $-C(O)C_6H_5$ | 4-Methoxybenzyl | 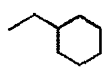 | $-CH_3$ |
| (1-40) | $-C(O)C_6H_5$ | 4-Methoxybenzyl | 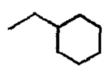 | $-C_2H_5$ |

Among the α-substituted-β-amino acid esters shown in Table 1, (1-1), (1-2), (1-9), (1-10), (1-17), (1-18), (1-25), (1-26), (1-33) and (1-34) are preferred, (1-9), (1-10), (1-17), (1-18), (1-25) and (1-26) are particularly preferred, and further preferred is one in which X is an oxygen atom.

The optical isomer mixture of the α-substituted-β-amino acid ester (1) can be obtained by a known production method. For example, this production method is described in ARKICOV 2010 (iX), pages 196 to 205 and so on.

The optical isomer mixture of the α-substituted-β-amino acid ester (1) used in the present production method may be a racemate, or an isomer mixture excessively containing one of optical isomers. These racemate and isomer mixture may be one newly prepared, or an isomer mixture excessively containing one of optical isomers prepared by optically resolving the racemate.

The enzyme capable of asymmetrically hydrolyzing the ester site ($-CO_2R^4$ group) of the optical isomer mixture of the α-substituted-β-amino acid ester (1) can be a hydrolase originated from *Chromobacterium spp.* microorganism, *Mucor spp.* microorganism, or the like, further preferably a hydrolase originated from *Chromobacterium chocolatum*

microorganism or *Mucor miehei* microorganism.

Specific examples of the enzyme capable of asymmetrically hydrolyzing the ester site of the optical isomer mixture of the α-substituted-β-amino acid ester (1) include an esterase or lipase derived from *Chromobacterium* SC-YM-1 strain (Agency of Industrial Science and Technology: National Institute of Bioscience and Human Technology, accession number: FERM BP-6703) and CHIRAZYME L-9 [derived from *Mucor miehei*, CHIRAZYME L-9 (trade name)] that is a commercially available enzyme.

The enzyme capable of asymmetrically hydrolyzing the ester site of the optical isomer mixture of the α-substituted-β-amino acid ester (1) (hereinafter, referred to as "the present enzyme" in some cases) may be an enzyme derived from a mutant induced from these microorganisms by treatment with a mutagen, ultraviolet irradiation or the like, an enzyme produced by a recombinant microorganism into which a gene encoding the present enzyme of these microorganisms is introduced and transformed, or a variant-type enzyme obtained by deletion, addition or substitution of 1 or 2 of specific amino acids in the amino acid sequence of the present enzyme by a genetic engineering technique, and those capable of selectively hydrolyzing the ester site of the optical isomer mixture of the α-substituted-β-amino acid ester (1) can be used for the production method of the present invention.

Examples of a method for preparing a recombinant microorganism into which a gene encoding the present enzyme is introduced and transformed include a method in accordance with a normal genetic engineering technique described in Molecular Cloning 2nd edition, published by Cold Spring Harbor Laboratory, 1989, edited by J.Sambrook, E.F.Fritsch, and T.Maniatis; and the like. Further specifically, examples thereof include a method in accordance with the method described in JP 3875283. Examples of the present enzyme produced as above include an esterase derived from *Chromobacterium* SC-YM-1 strain (FERM BP-6703) (JP Patent 3875283).

In addition, examples of a method for producing a variant-type enzyme by a genetic engineering technique include a method of Olfert Landt et al. (Gene, 96, 125-128, 1990). Further specifically, examples thereof include a method in accordance with the method described in JP 2000-78988A and a method in accordance with the method described in JP Patent 3486942. Examples of the variant-type enzyme that can be prepared as above include variant-type esterase and lipase prepared from an esterase derived from *Chromobacterium* SC-YM-1 strain.

The present enzyme used in the present production method may be a variant-type enzyme obtained by deletion, addition or substitution of 1 or 2 of amino acids constituting the hydrolase originated from *Chromobacterium spp.* microorganism. In order to prepare the variant-type enzyme, a genetic engineering technique, specifically, a site-specific mutagenesis of a gene is used.

Particularly preferred examples of the present enzyme derived from *Chromobacterium* SC-YM-1 strain described above include enzymes comprising an amino acid sequence encoded by any DNA of the following (a), (b), (c), (d), (e) or (f).

(a) Nucleotide sequence shown in SEQ ID NO: 1.
(b) Nucleotide sequence encoding an amino acid sequence formed by, in the amino acid sequence of the nucleotide sequence shown in SEQ ID NO: 1, substituting the 160th amino acid with the amino acid selected from the following Group A, and substituting the 189th amino acid with the amino acid selected from the following Group B.
(c) Nucleotide sequence that is a nucleotide sequence of DNA hybridizing with DNA comprising the above (b) under stringent conditions, and encodes an amino acid sequence of an enzyme having a catalytic function equivalent to that of an enzyme comprising an amino acid sequence encoded by the above (b).
(Group A)
Alanine, valine, leucine, isoleucine and serine
(Group B)
Alanine, valine, leucine, isoleucine, serine, threonine, phenylalanine, histidine, tyrosine and arginine
(d) Amino acid sequence wherein the 325th amino acid is substituted with isoleucine, in the amino acid sequence shown in SEQ ID NO: 1.
(e) Amino acid sequence wherein the 240th amino acid is substituted with alanine and the 288th amino acid is substituted with alanine, in the amino acid sequence shown in SEQ ID NO: 1.
(f) Amino acid sequence wherein the 43rd amino acid is substituted with serine, in the amino acid sequence shown in SEQ ID NO: 1.

Further preferred examples of the enzyme derived from *Chromobacterium* SC-YM-1 described above include esterase 160A189Y363term derived from *Chromobacterium* SC-YM-1 strain, esterase 160S189F363term derived from *Chromobacterium* SC-YM-1 strain, esterase 160A189F363term derived from *Chromobacterium* SC-YM-1 strain, esterase V325I derived from *Chromobacterium* SC-YM-1 strain, esterase T240AV288A derived from *Chromobacterium* SC-YM-1 strain, and esterase N43SA363term derived from *Chromobacterium* SC-YM-1 strain.

The site-specific mutagenesis will be briefly described. First, a gene encoding a variant-type enzyme (hereinafter, referred to as "gene 1" in some cases) is obtained. In order to obtain this gene 1, a wild-type gene encoding a

hydrolase originated from *Chromobacterium spp.* microorganism (hereinafter, referred to as "wild-type gene 1" in some cases) should be obtained. The wild-type gene can be obtained from *Chromobacterium* SC-YM-1 strain in accordance with, for example, a normal genetic engineering technique described in Molecular Cloning 2nd edition, published by Cold Spring Harbor Laboratory, 1989, edited by J.Sambrook, E.F.Fritsch, T.Maniatis, and the like. That is, *Chromobacterium* SC-YM-1 strain is cultured using, for example, LB medium (triptone 1.0%, yeast extract 0.5%, NaCl 0.5%), and the obtained microbial cells are disrupted by a method such as ultrasonic crushing, and subjected to protease treatment or the like, and then genomic DNA is extracted. The obtained genomic DNA is cleaved with an appropriate restriction enzyme, and inserted into λ-gt11 as a pharge vector, pUC19 as a plasmid vector or the like, using ligase to prepare genomic DNA library. The genomic DNA library is screened by, for example, a hybridization method using a synthetic DNA probe corresponding to a part of the amino acid sequence of the wild-type enzyme, or a screening method such as a method of determining the activity of the wild-type enzyme, and a clone containing the wild-type gene 1 can be obtained.

Site-specific variation is introduced into the wild-type gene 1, whereby the gene 1 can be prepared. Examples of the site-specific mutagenesis include methods suggested by Olfert Landt et al. (Gene, 96, 125-128, 1990), Smith et al. (Genetic Engineering 31 Setlow, J. and Hollaender, A Plenum: New York), Vlasuk et al. (Experimental Manipulation of Gene Expression, Inouye, M. : Academic Press, New York), and Hos.N.Hunt et al. (Gene, 77, 51, 1989); and the like. For example, in order to prepare gene 1 encoding an amino acid sequence in which specific amino acid is substituted with other amino acid, in an amino acid sequence of a hydrolase originated from *Chromobacterium spp.* microorganism using the method of Olfert Landt et al. (Gene, 96, 125-128, 1990), first, a plasmid DNA into which wild-type gene 1 is incorporated is prepared in accordance with the method described in Molecular Cloning 2nd edition, published by Cold Spring Harbor Laboratory, 1989, edited by J. Sambrook, E. F. Fritsch, T.Maniatis, and the like. Subsequently, a DNA fragment should be amplified by PCR method, for example, using oligonucleotide containing a nucleotide sequence in which specific amino acid is substituted as one primer and using the obtained plasmid DNA as a template. Here, as the conditions of PCR reaction, for example, after incubation at 94°C for 5 minutes, a treatment of incubation at 94°C for 1 minute, and then at 50°C for 2 minutes, and further at 75°C for 3 minutes is performed 20 cycles, and the mixture is lastly incubated at 75°C for 8 minutes. The DNA fragment amplified as above is digested, for example, with restriction enzymes BstPI and XbaI, and subjected to ligation reaction with a plasmid DNA containing wild-type gene 1 subjected to the same restriction enzyme digestion, whereby intended gene 1 can be obtained.

Using the gene 1 prepared as above, a variant-type enzyme can be produced and obtained in accordance with a normal genetic engineering technique. Specifically, for example, a plasmid capable of expressing the gene 1 in a host microorganism cell should be prepared and introduced into a host microorganism cell to transform the host microorganism cell, and the transformant microorganism should be cultured. The plasmid as described above can preferably include a plasmid in which the gene 1 is introduced into an expression vector having a promoter and a detectable marker, which is replicable in a host microorganism cell, and is easily isolated and purified from the host. As the expression vector, various commercially available vectors can be used, and for example, an expression vector containing a promoter such as lac, trp and tac (available from Pharmacia Biotech Inc., and the like) can be used for the expression in *E. coli.*

As the host microorganism cell, either of eukaryote or prokaryote, for example, *E. coli* can be used. The plasmid described above can be introduced into the host microorganism cell to transform the host microorganism cell in accordance with a normal genetic engineering technique. Microorganisms having the plasmid containing the gene 1 obtained as above can be cultured by a conventional method. When a host microorganism cell is, for example, *E. coli,* culture is carried out in a culture medium appropriately containing suitable trace nutrients such as carbon source, nitrogen source and vitamins. As the culture method, either of a solid culture or a liquid culture is available, and preferable method is an aeration stirring culture. Collection of a variant-type enzyme from the cultured microbial cells can include a normal protein isolation and purification method, specifically, the method already shown as the method of purifying an enzyme from a culture solution.

In the present invention, a mutation primer is prepared such that the 160th amino acid is substituted with the amino acid selected from the Group A, and the 189th amino acid is substituted with the amino acid other than glycine, in the amino acid sequence of the nucleotide sequence shown in SEQ ID NO: 1, and amplification by PCR method should be performed. Preferably, specific mutation is introduced such that the 160th amino acid is substituted with the amino acid selected from the Group A, and the 189th amino acid is substituted with the amino acid selected from the Group B.

Here, site-specific variation may be simultaneously introduced into the 160th and 189th amino acids in the amino acid sequence of the nucleotide sequence shown in SEQ ID NO: 1.

In addition, a mutation primer in which the 325th amino acid is substituted with isoleucine in the amino acid sequence of the nucleotide sequence shown in SEQ ID NO: 1, a mutation primer in which the 240th amino acid is substituted with alanine and the 288th amino acid is substituted with alanine in the amino acid sequence shown in SEQ ID NO: 1, or a mutation primer in which the 43rd amino acid is substituted with serine in the amino acid sequence shown in SEQ ID NO: 1 is prepared, and amplification by PCR method should be performed.

Next, a hydrolase originated from *Mucor spp.* microorganism will be described. Among hydrolases originated from the *Mucor spp.* microorganism, a hydrolase originated from *Mucor miehei* microorganism is preferred. In addition, a commercially available enzyme can be also used as the hydrolase originated from *Mucor miehei* microorganism. The commercially available enzyme is CHIRAZYME L-9 [manufactured by Roche Diagnostics] or the like. Since the commercially available enzyme is easily available, it is preferred in that the present production method can be easily carried out. Here, a variant-type enzyme obtained by deletion, addition or substitution of 1 or 2 of amino acids constituting the hydrolase is also prepared in the hydrolase originated from *Mucor spp.* microorganism, and then the variant-type enzyme can be used in the present production method. The site-specific mutagenesis described as a method for preparing a variant-type enzyme from the hydrolase originated from *Chromobacterium spp.* microorganism can also be used in the preparation of the variant-type enzyme in this case.

Microorganism producing the present enzyme can be all liquid-cultured by a conventional method. As a culture medium, various culture media appropriately containing a carbon source, a nitrogen source, a mineral and the like used in usual microorganism culture can be used. There can be used as the carbon source, for example, glucose, glycerol, starch, dextrin, an organic acid, molasses and the like; and as the nitrogen source, fats and oils, polypeptone, yeast extract, meat extract, malt extract, soybean flour, corn steep liquor, cottonseed flour, dried yeast, casamino acid, ammonium chloride, ammonium nitrate, ammonium sulfate, urea and the like; and as the mineral, for example, salts, sulfates, acetates, carbonates and phosphates of potassium, sodium, magnesium, calcium, iron, manganese, cobalt, zinc, copper and the like, specifically, potassium chloride, sodium chloride, magnesium sulfate, ferrous sulfate, manganese sulfate, cobalt chloride, zinc sulfate, copper sulfate, sodium acetate, calcium carbonate, sodium carbonate, potassium phosphate, monopotassium dihydrogen phosphate, dipotassium hydrogen phosphate, sodium phosphate, monosodium dihydrogen phosphate, disodium hydrogen phosphate and the like. In addition, in order to enhance the asymmetric hydrolysis ability of the above-described microorganisms to the $\alpha$-substituted-$\beta$-amino acid ester (1), a triglyceride such as an olive oil or tributyrin or the $\alpha$-substituted-$\beta$-amino acid ester (1) may be appropriately added to the medium.

Culture is preferably aerobically carried out in general, and a shaking culture or an aeration stirring culture is suitable. The culture temperature is usually 20 to 40°C and preferably 25 to 35°C, and the pH is preferably 6 to 8. The culturing time depends on various conditions, and it is preferably 1 to 7 days.

In addition, it is also possible to appropriately adopt a solid culture method as necessary, as long as it is a method capable of obtaining a microbial cell having asymmetric hydrolysis ability of the $\alpha$-substituted-$\beta$-amino acid ester (1).

In order to purify the present enzyme from a microorganism culture cultured as described above, it should be usually purified in accordance with the method used in the general purification of an enzyme. For example, first, the microbial cells in the microorganism culture are crushed by a method such as sonication, dynomill treatment or French press treatment. The insoluble material is removed from the obtained crushed liquid by centrifugation or the like, and then the target enzyme can be purified by one or suitably combining a plurality of cation exchange column chromatography, anion exchange column chromatography, hydrophobic column chromatography, gel filtration column chromatography and the like normally used in the purification of an enzyme. Examples of the carrier used in the column chromatography include DEAE-Sepharose fastflow (manufactured by Amersham Pharmacia Biotech Inc.), and Butyl-Toyopearl 650S (manufactured by Tosoh Corporation).

The present enzyme can be used in various forms such as a purified enzyme, a crude enzyme, a microorganism culture, a microbial cell and a treated product thereof. The treated product used herein refers to, for example, freeze-dried microbial cells, acetone dried microbial cells, microbial cell ground material, autolysates of microbial cells, sonicates of microbial cells, microbial cell extracts, alkali-treated products of microbial cells, or the like. Further, the enzymes having various purities or forms described above may also be used after being immobilized in accordance with a known method such as an adsorption method onto an inorganic carrier such as silica gel or ceramics, cellulose, ion exchange resin or the like, a polyacrylamide method, a sulfur-containing polysaccharide gel method (for example, a carrageenan gel method), an alginic acid gel method, or an agar gel method.

Subsequently, the present production method using a enzyme selected from the group consisting of the hydrolase and variant-type enzymes thereof, a culture of a microorganism capable of producing the enzyme or a treated product thereof (hereinafter, the enzyme, culture solution and treated product used herein collectively referred to as "the present enzyme and the like") will be described.

The amount of the present enzyme and the like used is appropriately selected so as not to cause a delay in reaction time and a decrease in selectivity of asymmetrical hydrolysis. When a purified enzyme, a crude enzyme or a commercially available enzyme is used, for example, the amount used is usually 0.001 to 2 times by weight and preferably 0.002 to 0.5 times by weight based on the optical isomer mixture of the $\alpha$-substituted-$\beta$-amino acid ester (1) in terms of the weight of the enzyme contained in the present enzyme and the like, and when a microorganism culture, microbial cells or a treated product thereof is used, the amount used is usually 0.01 to 200 times by weight and preferably 0.1 to 50 times by weight based on the optical isomer mixture of the $\alpha$-substituted-$\beta$-amino acid ester (1) in terms of the weight of the enzyme contained in the present enzyme and the like.

The water used in the asymmetrical hydrolysis reaction may be an aqueous buffer solution. Examples of the aqueous buffer solution include aqueous buffer solutions of an inorganic acid salt such as aqueous solutions of an alkali metal phosphate, for example, an aqueous solution of sodium phosphate and an aqueous solution of potassium phosphate; aqueous buffer solutions of an organic acid salt such as aqueous solutions of an alkali metal acetate, for example, an aqueous solution of sodium acetate and an aqueous solution of potassium acetate; and the like. The amount of the water or aqueous buffer solution used should be usually 0.5 times by weight or more based on the weight of the optical isomer mixture of the α-substituted-β-amino acid ester (1), and may be the amount which is the same as that of the solvent in some cases, but is usually 200 times by weight or less.

The asymmetrical hydrolysis reaction may be carried out in an organic solvent such as a hydrophobic organic solvent or a hydrophilic organic solvent. Examples of the hydrophobic organic solvent include ethers such as tert-butyl methyl ether and isopropyl ether; and hydrocarbons such as toluene, hexane, cyclohexane, heptane, octane and isooctane. Examples of the hydrophilic organic solvent include alcohols such as tert-butyl alcohol, methanol, ethanol, isopropyl alcohol, isobutyl alcohol and n-butyl alcohol; ethers such as tetrahydrofuran; sulfoxides such as dimethyl sulfoxide; ketones such as acetone; nitriles such as acetonitrile; and amides such as N,N-dimethylformamide. These hydrophobic organic solvents and hydrophilic organic solvents are used alone or in combination of two or more kinds, and the hydrophobic organic solvent and the hydrophilic organic solvent may be used in combination. In addition, in the present production method, water or an aqueous buffer solution, a hydrophobic organic solvent and a hydrophilic organic solvent may be mixed and used.

When the organic solvent is used, the amount used is usually 200 times by weight or less and preferably within the range of 0.1 to 100 times by weight based on the weight of the optical isomer mixture of the α-substituted-β-amino acid ester (1).

The asymmetrical hydrolysis reaction is carried out, for example, by a method of mixing the optical isomer mixture of the α-substituted-β-amino acid ester (1) and the present enzyme and the like, and when an organic solvent is used, the organic solvent, water, the optical isomer mixture of the α-substituted-β-amino acid ester (1), the present enzyme and the like should be mixed.

As the pH of the reaction system, the value in which the asymmetrical hydrolysis by the present enzyme and the like proceeds with high selectivity is appropriately selected, and is not particularly limited, but is usually in the range of a pH of 4 to 10, and preferably a pH of 6 to 8. During the reaction, an acid or a base may be added to adjust the pH within the appropriately selected range. Examples of the acid include hydrogen chloride, hydrogen bromide, phosphoric acid, sulfuric acid, nitric acid and salts thereof; organic acids such as acetic acid, citric acid, and methanesulfonic acid and salts thereof. Examples of the base include alkali metal hydroxides such as sodium hydroxide and potassium hydroxide; alkali metals and alkaline earth metal carbonates such as sodium carbonate, potassium carbonate and calcium carbonate; alkali metal bicarbonates such as sodium bicarbonate and potassium bicarbonate; phosphates such as sodium dihydrogen phosphate, disodium hydrogen phosphate, potassium dihydrogen phosphate and dipotassium hydrogen phosphate; organic bases such as diethylamine, diisopropylethylamine, triethylamine, cyclohexylamine, benzylamine, phenethylamine and pyridine; and ammonia. The bases may be used alone or in a mixture of two or more kinds. These are selected depending on the kind of the present enzyme and the like used in the present production method, and such that hydrolysis of the α-substituted-β-amino acid ester (1) is not caused by the action of the added acid or base. The acid or base is usually used in the form of an aqueous solution, but when an organic solvent is used in the reaction, it may be used in the form of an organic solvent or a mixed solution of an organic solvent and water. As the organic solvent, those which are the same as those used in the reaction can be used. In addition, water dissolving the acid or base may be replaced by an aqueous buffer solution. Furthermore, the acid or base may be used as a solid or in the state suspended in a solution.

As the reaction temperature, the temperature in which the asymmetrical hydrolysis by the present enzyme and the like proceeds with high selectivity is appropriately selected, and is not particularly limited, but the stability of the present enzyme in the present enzyme and the like tends to decrease when the temperature is too high, and the reaction rate tends to decrease when the temperature is too low. On the other hand, the lower the temperature is, the more the selectivity tends to increase. The reaction temperature is usually within the range of -10 to 65°C, and preferably within the range of -5 to 50°C.

The reaction time is usually selected from within the range of 1 to 100 hours, and preferably selected from within the range of 1 to 50 hours. The reaction solution in the reaction system of the present production method is appropriately sampled, the degree of disappearance of the α-substituted-β-amino acid ester (1) or the degree of production of the α-substituted-β-amino acid (2) is determined by a suitable analytical means such as HPLC or GC, and then the reaction time can also be set.

Thus, a solution of the optically-active α-substituted-β-amino acid (2) can be obtained, and usually, a post-treatment operation is further carried out in order to separate from the enzyme and buffer used in the reaction or the carboxylic acid generated by the hydrolysis reaction.

Examples of the post-treatment include a method of distilling off the solvent in the reaction solution and then

separating and purifying the α-substituted-β-amino acid (2) using silica gel chromatography; a method of distilling off the solvent in the same manner and then separating and purifying the α-substituted-β-amino acid (2) by distillation; and a method of separating and purifying the α-substituted-β-amino acid (2) by separation operation.

In separating and purifying the α-substituted-β-amino acid (2) by separation operation, when an organic solvent that dissolves in both water and a hydrophobic organic solvent is used during the reaction, this organic solvent may be removed by distillation off and then used. In addition, when an insoluble enzyme, an immobilized carrier and the like are present in the solution, these may be removed by filtration.

In the reaction solution after the completion of the reaction of the present production method are contained the α-substituted-β-amino acid (2) as a product of the asymmetrical hydrolysis reaction and the remaining α-substituted-β-amino acid ester (1). In order to separate both of them, for example, a method of carrying out an extraction operation by water/hydrophobic organic solvent to distribute the remaining α-substituted-β-amino acid ester (1) and the α-substituted-β-amino acid (2) to the organic layer (hydrophobic organic solvent layer) and the aqueous layer, respectively, and separating the organic layer and the aqueous layer is adopted.

In order to separate the target optically-active α-substituted-β-amino acid (2) from the present enzyme and the like, the buffer and other water-soluble components, the optically-active α-substituted-β-amino acid (2) should be extracted to the organic layer using a hydrophobic organic solvent and separated from the aqueous layer.

Examples of the hydrophobic organic solvent include ethers such as tert-butyl methyl ether and isopropyl ether; hydrocarbons such as toluene, hexane, cyclohexane, heptane, octane and isooctane; halogenated hydrocarbons such as dichloromethane, dichloroethane, chloroform, chlorobenzene and ortho-dichlorobenzene; and esters such as ethyl acetate, methyl acetate and butyl acetate. In a case where these hydrophobic organic solvents are used during the reaction, when the reaction solution after the completion of the reaction is separated into the organic layer and the aqueous layer, the separation operation can also be carried out as it is. Further, in a case where the hydrophobic organic solvent is not used during the reaction, a case where the reaction solution is not easily separated into the organic layer and the aqueous layer since the amount of the hydrophobic organic solvent or water used is low, or a case where the reaction solution cannot be easily separated due to the low amount of water used, the reaction solution should be separated after appropriately adding the hydrophobic organic solvent, water or the like. The amount of the hydrophobic organic solvent used is not particularly limited, but is usually 0.1 to 200 times by weight, and preferably 0.2 to 100 times by weight, based on the weight of the optical isomer of the α-substituted-β-amino acid ester (1).

The pH during the extraction of the target substance is usually within the range of 2 to 10 and preferably within the range of 4 to 8. In order to adjust the pH, an acid or a base can also be appropriately used. Specific examples of the acid or base used herein are the same as those exemplified for adjusting the pH of the reaction system of the present production method. When the extraction of the target substance from the aqueous layer is insufficient, the same extraction and separation operation may be repeated multiple times. Similarly, when the removal of the water-soluble component from the organic layer is insufficient, the same extraction and separation operation may be repeated multiple times.

The remaining ester separated from carboxylic acid as an asymmetrical hydrolysate by the above extraction can be isolated by distilling off the organic solvent in the oil layer. The obtained optically-active α-substituted-β-amino acid ester (1) is subjected to racemization treatment, whereby it can be reused as an optical isomer mixture of the α-substituted-β-amino acid ester (1).

The unreacted α-substituted-β-amino acid ester (1) isolated by distilling off the organic solvent in the oil layer may be further purified by column chromatography or the like.

The optically-active α-substituted-β-amino acid (2) as an asymmetrical hydrolysate is contained in the aqueous layer after separation by the above extraction, and can be easily taken out from the aqueous layer by distillation off water, extraction using an organic solvent after neutralization treatment, or the like. The separated optically-active α-substituted-β-amino acid (2) can be isolated by distilling off the organic solvent in the oil layer.

The thus obtained optically-active α-substituted-β-amino acid (2) may be further purified by purification operation such as column chromatography, recrystallization, reprecipitation or the like. In addition, in the purification operation such as recrystallization or reprecipitation, the optically-active α-substituted-β-amino acid (2) is further made into a salt by a suitable base, and then this salt is purified by recrystallization or reprecipitation, and the purified salt can also be converted again to the optically-active α-substituted-β-amino acid (2) by an appropriate method.

Specific examples of the thus obtained optically-active α-substituted-β-amino acid (2) are shown in Table 2 by the combinations of $R^1$ to $R^3$ in formula (2).

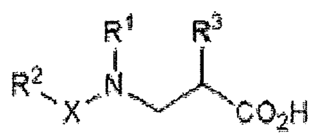

(2a)

[Table 2]

| α-substituted-β-amino acid | $R^1$ | $R^2$ | $R^3$ |
|---|---|---|---|
| (2-1) | -H | Benzyl | 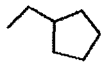 |
| (2-2) | -H | Benzyl | 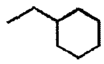 |
| (2-3) | -H | 4-Methoxybenzyl | 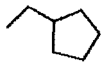 |
| (2-4) | -H | 4-Methoxybenzyl | 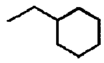 |
| (2-5) | -CHO | Benzyl | 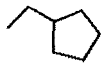 |
| (2-6) | -CHO | Benzyl | 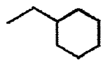 |
| (2-7) | -CHO | 4-Methoxybenzyl | 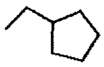 |
| (2-8) | -CHO | 4-Methoxybenzyl | 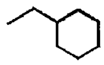 |
| (2-9) | $-C(O)CH_3$ | Benzyl | 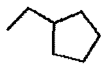 |
| (2-10) | $-C(O)CH_3$ | Benzyl | 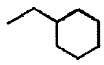 |
| (2-11) | $-C(O)CH_3$ | 4-Methoxybenzyl | 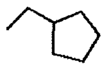 |
| (2-12) | $-C(O)CH_3$ | 4-Methoxybenzyl | 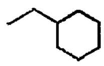 |
| (2-13) | $-C(O)C_2H_5$ | Benzyl | 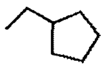 |
| (2-14) | $-C(O)C_2H_5$ | Benzyl | 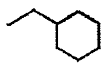 |
| (2-15) | $-C(O)C_2H_5$ | 4-Methoxybenzyl | 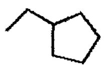 |
| (2-16) | $-C(O)C_2H_5$ | 4-Methoxybenzyl | 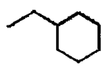 |
| (2-17) | $-C(O)C_6H_5$ | Benzyl | 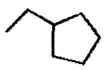 |
| (2-18) | $-C(O)C_6H_5$ | Benzyl | 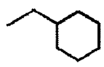 |
| (2-19) | $-C(O)C_6H_5$ | 4-Methoxybenzyl | 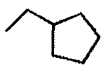 |

(continued)

| α-substituted-β-amino acid | $R^1$ | $R^2$ | $R^3$ |
|---|---|---|---|
| (2-20) | $-C(O)C_6H_5$ | 4-Methoxybenzyl | 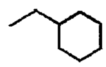 |

In the present invention, when an enzyme capable of selectively hydrolyzing the ester site of an R-form of the α-substituted-β-amino acid ester (1) is used as the present enzyme, the obtained optically-active α-substituted-β-amino acid (2) is rich in the R-form, and when an enzyme capable of selectively hydrolyzing the ester site of an S-form of the α-substituted-β-amino acid ester (1) is used, the obtained optically-active α-substituted-β-amino acid (2) is rich in the S-form. In addition, when the R-form of the α-substituted-β-amino acid (2) is obtained using the enzyme capable of selectively hydrolyzing the ester site of an R-form of the α-substituted-β-amino acid ester (1), the S-form of the α-substituted-β-amino acid ester (1) is obtained at the same time, and this can be separated from the R-form of the α-substituted-β-amino acid (2) by the above extraction, separation operation, and the like, and isolated.

EXAMPLES

Hereinbelow, the present invention will be described in more detail by way of examples and the like, but the present invention is not limited to these examples.

Examples 1 to 8

Production of optically-active 2-cyclopentylmethyl-3-[(N-benzyloxy-N-formyl)amino]propanoic acid (2-5)

Each of *E. coli* recombinant soluble fractions producing various enzymes or an enzyme shown in Table 3 was weighed into a vessel in the amount shown in Table 4, and 5 mL of a 0.1 M potassium phosphate buffer solution (pH 7.0), 40.0 mg of an optical isomer mixture (racemate) of 2-cyclopentylmethyl-3-[(N-benzyloxy-N-formyl)amino]propanoic acid methyl ester and 1 mL of tert-butyl methyl ether were added. This solution was stirred at 25°C for 48 hours, then 1 mL of an aqueous 3.4% phosphoric acid solution and 10 mL of tert-butyl methyl ether were added, and then mixed. The mixture was allowed to stand still, and then the tert-butyl methyl ether layer was analyzed for its optical purity by high performance liquid chromatography [column: CHIRALPAK AD-H, 4.6 mmϕ × 25 cm, 5 μm (manufactured by Daicel Corporation)] and for its chemical purity by high performance liquid chromatography [column: Cadenza CD-18, 4.6 mmϕ × 15 cm, 3 μm (manufactured by Imtakt)], and the conversion and enantiomeric excess of the resulting optically-active 2-cyclopentylmethyl-3-[(N-benzyloxy-N-formyl)amino]propanoic acid were determined. The results are shown in Table 4.

[Table 3]

| Examples | Name of Enzyme | Origin of Enzyme | Method for Preparing or Obtaining Enzyme |
|---|---|---|---|
| 1 | Wild Strain Esterase Originated from *Chromobacterium* SC-YM-1 strain | *Chromobacterium chocolatum* | Prepared According to Method Described in Example 7 of JP-3875283. |
| 2 | Esterase Originated from *Chromobacterium* SC-YM-1 strain, 160A189Y363term | *Chromobacterium chocolatum* | Prepared According to Method Described in Example 12 of JP-3486942. |
| 3 | Esterase Originated from *Chromobacterium* SC-YM-1 strain, 160S189F363term | *Chromobacterium chocolatum* | Prepared According to Method Described in Example 12 of JP-3486942. |
| 4 | Esterase Originated from *Chromobacterium* SC-YM-1 strain, 160A189F363term | *Chromobacterium chocolatum* | Prepared According to Method Described in Example 12 of JP-3486942. |
| 5 | Esterase Originated from *Chromobacterium* SC-YM-1 strain, V325I | *Chromobacterium chocolatum* | Prepared According to Method Described in Example 2 of JP 2000-78988A. |

(continued)

| Examples | Name of Enzyme | Origin of Enzyme | Method for Preparing or Obtaining Enzyme |
|---|---|---|---|
| 6 | Esterase Originated from *Chromobacterium* SC-YM-1 strain, T240AV288A | *Chromobacterium chocolatum* | Prepared According to Method Described in Example 2 of JP 2000-78988A. |
| 7 | Esterase Originated from *Chromobacterium* SC-YM-1 strain, N43SA363term | *Chromobacterium chocolatum* | Prepared According to Method Described in Example 2 of JP 2000-78988A. |
| 8 | CHIRAZYME L-9 | *Mucor miehei* | Manufactured by Roche Diagnostics |

[Table 4]

| Examples | Amount of Enzyme (mg) | Conversion Rate (%) | Enantiomeric Excess (%ee) | Excessive Optical Isomer |
|---|---|---|---|---|
| 1 | 206.4 | 17.6 | 99.9 | (R)-form |
| 2 | 200.0 | 25.9 | 98.9 | (R)-form |
| 3 | 201.8 | 15.1 | 99.3 | (R)-form |
| 4 | 200.7 | 11.8 | 100.0 | (R)-form |
| 5 | 201.0 | 18.1 | 99.9 | (R)-form |
| 6 | 208.2 | 17.6 | 99.2 | (R)-form |
| 7 | 202.1 | 18.7 | 100.0 | (R)-form |
| 8 | 2.0 | 13.3 | 99.5 | (R)-form |

Examples 9 to 16

Production of optically-active 2-cyclopentylmethyl-3-[(N-benzyloxy-N-formyl)amino]propanoic acid (2-5)

Each of *E. coli* recombinant soluble fractions producing various enzymes or an enzyme shown in Table 5 was weighed into a vessel in the amount shown in Table 6, and 5 mL of a 0.1 M potassium phosphate buffer solution (pH7.0) and 40.0 mg of an optical isomer mixture (racemate) of 2-cyclopentylmethyl-3-[(N-benzyloxy-N-formyl)amino]propanoic acid methyl ester were added. This solution was stirred at 25°C for 48 hours, and then 1 mL of an aqueous 3.4% phosphoric acid solution and 10 mL of tert-butyl methyl ether were added, and then mixed. The mixture was allowed to stand still, then the tert-butyl methyl ether layer was analyzed in the same manner as in Example 1, and the conversion and enantiomeric excess of the resulting optically-active 2-cyclopentylmethyl-3-[(N- benzyloxy-N-formyl)amino]propanoic acid were determined. The results are shown in Table 6.

[Table 5]

| Examples | Name of Enzyme | Origin of Enzyme | Method for Preparing or Obtaining Enzyme |
|---|---|---|---|
| 9 | Wild Strain Esterase Originated from *Chromobacterium* SC-YM-1 strain | *Chromobacterium chocolatum* | Prepared According to Method Described in Example 7 of JP-3875283. |
| 10 | Esterase Originated from *Chromobacterium* SC-YM-1 strain, 160A189Y363term | *Chromobacterium chocolatum* | Prepared According to Method Described in Example 12 of JP-3486942. |
| 11 | Esterase Originated from *Chromobacterium* SC-YM-1 strain, 160S189F363term | *Chromobacterium chocolatum* | Prepared According to Method Described in Example 12 of JP-3486942. |

(continued)

| Examples | Name of Enzyme | Origin of Enzyme | Method for Preparing or Obtaining Enzyme |
|---|---|---|---|
| 12 | Esterase Originated from *Chromobacterium* SC-YM-1 strain, 160A189F363term | *Chromobacterium chocolatum* | Prepared According to Method Described in Example 12 of JP-3486942. |
| 13 | Esterase Originated from *Chromobacterium* SC-YM-1 strain, V325I | *Chromobacterium chocolatum* | Prepared According to Method Described in Example 2 of JP 2000-78988A. |
| 14 | Esterase Originated from *Chromobacterium* SC-YM-1 strain, T240AV288A | *Chromobacterium chocolatum* | Prepared According to Method Described in Example 2 of JP 2000-78988A. |
| 15 | Esterase Originated from *Chromobacterium* SC-YM-1 strain, N43SA363term | *Chromobacterium chocolatum* | Prepared According to Method Described in Example 2 of JP 2000-78988A. |
| 16 | CHIRAZYME L-9 | *Mucor miehei* | Manufactured by Roche Diagnostics |

[Table 6]

| Examples | Amount of Enzyme (mg) | Conversion Rate (%) | Enantiomeric Excess (%ee) | Excessive Optical Isomer |
|---|---|---|---|---|
| 9 | 207.9 | 54.4 | 90.0 | (R)-form |
| 10 | 201.4 | 50.0 | 89.7 | (R)-form |
| 11 | 200.0 | 60.1 | 83.5 | (R)-form |
| 12 | 204.9 | 45.8 | 95.6 | (R)-form |
| 13 | 206.9 | 52.6 | 89.7 | (R)-form |
| 14 | 208.1 | 51.1 | 97.1 | (R)-form |
| 15 | 200.4 | 49.6 | 97.3 | (R)-form |
| 16 | 2.0 | 34.3 | 96.2 | (R)-form |

Example 17

Production of optically-active 2-cyclopentylmethyl-3-[(N-benzyloxy-N-formyl)amino]propanoic acid (2-5)

Into a vessel was weighed 50.3 g of a 0.1 M potassium phosphate buffer solution (pH 7.0), and 400.0 mg of an optical isomer mixture (racemate) of 2-cyclopentylmethyl-3-[(N-benzyloxy-N-formyl)amino]propanoic acid methyl ester, 7.4 g of tert-butyl methyl ether and 2.0 g of an alkali treated product of *E. coli* recombinant producing esterase N43SA363term derived from *Chromobacterium* SC-YM-1 strain were added thereto. This solution was stirred at 25°C for 158 hours, and then the mixture was analyzed in the same manner as in Example 1, and the conversion was found to be 44.2% (the enantiomeric excess of (R)-2-cyclopentylmethyl-3-[(N-benzyloxy-N-formyl)amino]propanoic acid: 100.0% ee). To the resulting reaction solution was added 10.6 g of an aqueous 3.4% phosphoric acid solution to have a pH of 5.0. To this solution was added 8.0 g of tert-butyl methyl ether, and the mixture was separated into the oil layer and the aqueous layer by separation. The aqueous layer was extracted twice with 8.0 g of tert-butyl methyl ether and subjected to a separation operation, and the aqueous layer was removed, and then the resulting mixture was combined with the oil layer previously obtained. The resulting mixed solution was filtered with celite, washed twice with 2.0 g of tert-butyl methyl ether, and then separated into the oil layer and the aqueous layer by phase separation. To the oil layer were added 250.5 mg of diisopropylethylamine and 4.0 g of water, and then the mixture was separated into the oil layer and the aqueous layer by phase separation. Subsequently, the aqueous layer was washed with 2.0 g of tert-butyl methyl ether, and the oil layer was removed. To the resulting aqueous layer were added 1.9 g of 1 N hydrochloric acid and 8.0 g of tert-butyl methyl ether, and the mixture was separated into the oil layer and the aqueous layer by separation. The

oil layer was dried over sodium sulfate, and then the organic solvent of the oil layer was distilled off under reduced pressure to obtain 134.6 mg of (R)-2-cyclopentylmethyl-3-[(N-benzyloxy-N-formyl)amino]propanoic acid as an oily substance. The resulting oily substance was analyzed in the same manner as in Example 1, and the yield of the resulting (R)-2-cyclopentylmethyl-3-[(N-benzyloxy-N-formyl)amino]propanoic acid was found to be 37.6%, and the enantiomeric excess thereof was found to be 100.0% ee.

Example 18

Production of optically-active 2-cyclopentylmethyl-3-[(N-benzyloxy-N-formyl)amino]propanoic acid (2-5)

Into a vessel was weighed 50.2 g of a 0.1 M potassium phosphate buffer solution (pH 7.0), and 400.0 mg of an optical isomer mixture (racemate) of 2-cyclopentylmethyl-3-[(N-benzyloxy-N-formyl)amino]propanoic acid ethyl ester, 7.4 g of tert-butyl methyl ether and 2.0 g of an alkali treated product of *E. coli* recombinant producing esterase 160A189Y363 term derived from *Chromobacterium* SC-YM-1 strain were added thereto. This solution was stirred at 25°C for 122 hours, and then the mixture was analyzed in the same manner as in Example 1, and the conversion was found to be 49.4% (the enantiomeric excess of (R)-2-cyclopentylmethyl-3-[(N-benzyloxy-N-formyl)amino]propanoic acid: 95.6% ee). To the resulting reaction solution was added 9.7 g of an aqueous 3.4% phosphoric acid solution to have a pH of 5.0. To this solution was added 8.0 g of tert-butyl methyl ether, and the mixture was separated into the oil layer and the aqueous layer by separation. The aqueous layer was extracted twice with 8.0 g of tert-butyl methyl ether and subjected to a separation operation, and the aqueous layer was removed, and then the resulting mixture was combined with the oil layer previously obtained. The resulting mixed solution was filtered with celite, and washed twice with 2.0 g of tert-butyl methyl ether, and then separated into the oil layer and the aqueous layer by phase separation. To the oil layer was added 4.0 g of a 1 N aqueous sodium hydroxide solution, and the mixture was separated into the oil layer and the aqueous layer by phase separation. Subsequently, the aqueous layer was washed with 4.0 g of tert-butyl methyl ether, and the oil layer was removed. To the resulting aqueous layer were added 3.9 g of 1 N hydrochloric acid and 8.0 g of tert-butyl methyl ether, and the mixture was separated into the oil layer and the aqueous layer by phase separation. The oil layer was washed with 0.8 g of 15% saline and dried over sodium sulfate, and then the organic solvent of the oil layer was distilled off under reduced pressure to obtain 170.6 mg of (R)-2-cyclopentylmethyl-3-[(N-benzyloxy-N-formyl)amino]propanoic acid as an oily substance. The resulting oily substance was analyzed in the same manner as in Example 1, and the yield of the resulting (R)-2-cyclopentylmethyl-3-[(N-benzyloxy-N-formyl) amino]propanoic acid was found to be 35.8%, and the enantiomeric excess thereof was found to be 95.2% ee.

Example 19

Production method of optically-active 2-cyclopentylmethyl-3-[(N-benzyloxy-N-formyl)amino]propanoic acid (2-5)

Into a vessel was weighed 3.0 g of an optical isomer mixture (racemate) of 2-cyclopentylmethyl-3-[(N-benzyloxy-N-formyl) amino] propanoic acid ethyl ester, and 14.2 g of a 0. 1 M potassium phosphate buffer solution (pH 7.5) and 0.75 g of an alkali treated product of *E. coli* recombinant producing esterase N43SA363term derived from *Chromobacterium* SC-YM-1 strain were added thereto. The mixture was stirred at 25°C for 24 hours, and then 7.2 g of a 0.1 M potassium phosphate buffer solution was added. The mixture was further stirred at 25°C for 53 hours, and then 24.4 g of a 0.1 M potassium phosphate buffer solution was added. The mixture was further stirred at 25°C for 26 hours, and then 45.0 g of a 0.1 M potassium phosphate buffer solution was added. The mixture was further stirred at 25°C for 80 hours, and then 120.0 g of a 0.1 M potassium phosphate buffer solution was added. This mixture was further stirred at 25°C for 96 hours, and then the mixture was analyzed in the same manner as in Example 1, and the conversion was found to be 46.3% (the enantiomeric excess of (R)-2-cyclopentylmethyl-3-[(N-benzyloxy-N-formyl)amino] propanoic acid: 99.8% ee). To the resulting reaction solution was added 2.3 g of an aqueous 85% phosphoric acid solution to have a pH of 5.0. To this solution was added 12. 0 g of tert-butyl methyl ether, and the mixture was separated into the oil layer and the aqueous layer by phase separation. The aqueous layer was extracted twice with 12.0 g of tert-butyl methyl ether and subjected to a separation operation, and the aqueous layer was removed, and then the resulting mixture was combined with the oil layer previously obtained. The resulting mixed solution was filtered with celite, and washed twice with 1.5 g of tert-butyl methyl ether, and then separated into the oil layer and the aqueous layer by phase separation. To the oil layer were added 1.74 g of diisopropylethylamine and 15. 0 g of water, and then the mixture was separated into the oil layer and the aqueous layer by phase separation. Subsequently, 6.0 g of water was added to the oil layer for extraction, the oil layer was removed, and then the resulting mixture was combined with the aqueous layer previously obtained. To the resulting aqueous layer were added 0.4 g of 35% hydrochloric acid and 9.0 g of tert-butyl methyl ether, and the mixture was separated into the oil layer and the aqueous layer by separation. The organic solvent of the oil layer was distilled off under reduced pressure to obtain 1.12 g of (R)-2-cyclopentylmethyl-3-[(N-benzyloxy-N-formyl)amino]propanoic acid as an oily substance. The resulting oily substance was analyzed in the same manner as in Example 1, and the yield of the resulting (R)-2-cyclopentylmethyl-3-[(N-benzyloxy-N-formyl) amino]propanoic acid was found to be 39.7%, and the enantiomeric excess thereof was found to be 99.5% ee.

Example 20

Production method of optically-active 2-cyclopentylmethyl-3-[(N-benzyloxy)amino]propanoic acid (2-1)

Into a vessel was weighed 21.0 g of a 0.1 M potassium phosphate buffer solution (pH 7.0), and 300.0 mg of an optical isomer mixture (racemate) of 2-cyclopentylmethyl-3-[(N-benzyloxy)amino]propanoic acid ethyl ester and 75.0 mg of an alkali treated product of *E. coli* recombinant producing esterase N43SA363term derived from *Chromobacterium* SC-YM-1 strain were added thereto. This solution was stirred at 25°C for 96 hours, and then 0.24 g of an aqueous 85% phosphoric acid solution was added to have a pH of 5.0. To this solution was added 6.0 g of tert-butyl methyl ether, and the mixture was separated into the oil layer and the aqueous layer by phase separation. The aqueous layer was extracted twice with 6.0 g of tert-butyl methyl ether and subjected to a separation operation, and the aqueous layer was removed, then the resulting mixture was combined with the oil layer previously obtained. The resulting mixed solution was filtered with celite, and washed twice with 1.5 g of tert-butyl methyl ether, and then separated into the oil layer and the aqueous layer by phase separation. To the oil layer were added 189.9 mg of diisopropylethylamine and 3.0 g of water, and then the mixture was separated into the oil layer and the aqueous layer by phase separation. Subsequently, the aqueous layer was washed with 1.5 g of tert-butyl methyl ether, and the oil layer was removed. To the resulting aqueous layer were added 1.6 g of 1 N hydrochloric acid and 6.0 g of tert-butyl methyl ether, and the mixture was separated into the oil layer and the aqueous layer by phase separation. The oil layer was dried over sodium sulfate, and then the organic solvent of the oil layer was distilled off under reduced pressure to obtain 17.7 mg of (R)-2-cyclopentylmethyl-3-[(N-benzyloxy)amino]propanoic acid as an oily substance. The resulting oily substance was analyzed in the same manner as in Example 1, and the yield of the resulting (R)-2-cyclopentylmethyl-3-[(N-benzyloxy)amino]propanoic acid was found to be 5.8%, and the enantiomeric excess thereof was found to be 99.2% ee.

Examples 21 to 27

Production of optically-active 2-cyclopentylmethyl-3-[(N-benzyloxy-N-acetyl)amino]propanoic acid (2-9)

Each of *E. coli* recombinant soluble fractions producing various enzymes shown in Table 7 was weighed into a vessel in the amount shown in Table 8, and 5 mL of a 0.1 M potassium phosphate buffer solution (pH 7.0) and 40.0 mg of an optical isomer mixture (racemate) of 2-cyclopentylmethyl-3-[(N-benzyloxy-N-acetyl) amino]propanoic acid ethyl ester were added. This solution was stirred at 25°C for 48 hours, then 1 mL of an aqueous 3.4% phosphoric acid solution and 10 mL of tert-butyl methyl ether were added, and then mixed. The mixture was allowed to stand still, and then the tert-butyl methyl ether layer was analyzed in the same manner as in Example 1, and the conversion and enantiomeric excess of the resulting optically-active 2-cyclopentylmethyl-3-[(N-benzyloxy-N-acetyl)amino]propanoic acid were determined. The results are shown in Table 8.

[Table 7]

| Examples | Name of Enzyme | Origin of Enzyme | Method for Preparing or Obtaining Enzyme |
|---|---|---|---|
| 21 | Wild Strain Esterase Originated from *Chromobacterium* SC-YM-1 strain | *Chromobacterium chocolatum* | Prepared According to Method Described in Example 7 of JP-3875283. |
| 22 | Esterase Originated from *Chromobacterium* SC-YM-1 strain, 160A189Y363term | *Chromobacterium chocolatum* | Prepared According to Method Described in Example 12 of JP-3486942. |
| 23 | Esterase Originated from *Chromobacterium* SC-YM-1 strain, 160S189F363term | *Chromobacterium chocolatum* | Prepared According to Method Described in Example 12 of JP-3486942. |
| 24 | Esterase Originated from *Chromobacterium* SC-YM-1 strain, 160A189F363term | *Chromobacterium chocolatum* | Prepared According to Method Described in Example 12 of JP-3486942. |

(continued)

| Examples | Name of Enzyme | Origin of Enzyme | Method for Preparing or Obtaining Enzyme |
|---|---|---|---|
| 25 | Esterase Originated from *Chromobacterium* SC-YM-1 strain, V325I | *Chromobacterium chocolatum* | Prepared According to Method Described in Example 2 of JP 2000-78988A. |
| 26 | Esterase Originated from *Chromobacterium* SC-YM-1 strain, T240AV288A | *Chromobacterium chocolatum* | Prepared According to Method Described in Example 2 of JP 2000-78988A. |
| 27 | Esterase Originated from *Chromobacterium* SC-YM-1 strain, N43SA363term | *Chromobacterium chocolatum* | Prepared According to Method Described in Example 2 of JP 2000-78988R. |

[Table 8]

| Examples | Amount of Enzyme (mg) | Conversion Rate (%) | Enantiomeric Excess (%ee) |
|---|---|---|---|
| 21 | 199.9 | 49.5 | 99.5 |
| 22 | 201.8 | 49.9 | 99.3 |
| 23 | 201.1 | 49.3 | 99.6 |
| 24 | 203.4 | 49.1 | 99.7 |
| 25 | 201.2 | 49.7 | 99.6 |
| 26 | 201.4 | 49.4 | 99.8 |
| 27 | 201.7 | 49.7 | 100.0 |

Examples 28 to 34

Production of optically-active 2-cyclopentylmethyl-3-[(N-benzyloxy-N-propionyl)amino]propanoic acid (2-13)

Each of *E. coli* recombinant soluble fractions producing various enzymes shown in Table 9 was weighed into a vessel in the amount shown in Table 10, and 5 mL of a 0.1 M potassium phosphate buffer solution (pH 7.0) and 40.0 mg of an optical isomer mixture (racemate) of 2-cyclopentylmethyl-3-[(N-benzyloxy-N-propionyl) amino]propanoic acid ethyl ester were added. This solution was stirred at 25°C for 48 hours, then 1 mL of an aqueous 3.4% phosphoric acid solution and 10 mL of tert-butyl methyl ether were added, and then mixed. The mixture was allowed to stand still, and then the tert-butyl methyl ether layer was analyzed in the same manner as in Example 1, and the conversion and enantiomeric excess of the resulting optically-active 2-cyclopentylmethyl-3-[(N-benzyloxy-N-propionyl)amino]propanoic acid were determined. The results are shown in Table 10.

[Table 9]

| Examples | Name of Enzyme | Origin of Enzyme | Method for Preparing or Obtaining Enzyme |
|---|---|---|---|
| 28 | Wild Strain Esterase Originated from *Chromobacterium* SC-YM-1 strain | *Chromobacterium chocolatum* | Prepared According to Method Described in Example 7 of JP-3875283. |
| 29 | Esterase Originated from *Chromobacterium* SC-YM-1 strain, 160A189Y363term | *Chromobacterium chocolatum* | Prepared According to Method Described in Example 12 of JP-3486942. |
| 30 | Esterase Originated from *Chromobacterium* SC-YM-1 strain, 160S189F363term | *Chromobacterium chocolatum* | Prepared According to Method Described in Example 12 of JP-3486942. |

(continued)

| Examples | Name of Enzyme | Origin of Enzyme | Method for Preparing or Obtaining Enzyme |
|---|---|---|---|
| 31 | Esterase Originated from *Chromobacterium* SC-YM-1 strain, 160A189F363term | *Chromobacterium chocolatum* | Prepared According to Method Described in Example 12 of JP-3486942. |
| 32 | Esterase Originated from *Chromobacterium* SC-YM-1 strain, V325I | *Chromobacterium chocolatum* | Prepared According to Method Described in Example 2 of JP 2000-78988A. |
| 33 | Esterase Originated from *Chromobacterium* SC-YM-1 strain, T240AV288A | *Chromobacterium chocolatum* | Prepared According to Method Described in Example 2 of JP 2000-78988A. |
| 34 | Esterase Originated from *Chromobacterium* SC-YM-1 strain, N43SA363term | *Chromobacterium chocolatum* | Prepared According to Method Described in Example 2 of JP 2000-78988A. |

[Table 10]

| Examples | Amount of Enzyme (mg) | Conversion Rate (%) | Enantiomeric Excess (%ee) |
|---|---|---|---|
| 28 | 200.6 | 44.1 | 100.0 |
| 29 | 201.4 | 46.0 | 100.0 |
| 30 | 200.7 | 39.9 | 100.0 |
| 31 | 203.1 | 33.7 | 100.0 |
| 32 | 199.0 | 43.1 | 100.0 |
| 33 | 199.0 | 43.5 | 100.0 |
| 34 | 201.0 | 48.5 | 100.0 |

Examples 35 to 41

Production of optically-active 2-cyclopentylmethyl-3-[(N-benzyloxy-N-benzoyl)amino]propanoic acid (2-17)

Each of *E. coli* recombinant soluble fractions producing various enzymes shown in Table 11 was weighed into a vessel in the amount shown in Table 12, and 5 mL of a 0.1 M potassium phosphate buffer solution (pH 7.0) and 40.0 mg of an optical isomer mixture (racemate) of 2-cyclopentylmethyl-3-[(N-benzyloxy-N-benzoyl) amino]propanoic acid ethyl ester were added. This solution was stirred at 25°C for 48 hours, then 1 mL of an aqueous 3.4% phosphoric acid solution and 10 mL of tert-butyl methyl ether were added, and then mixed. The mixture was allowed to stand still, and then the tert-butyl methyl ether layer was analyzed in the same manner as in Example 1, and the conversion and enantiomeric excess of the resulting optically-active 2-cyclopentylmethyl-3-[(N-benzyloxy-N-benzoyl)amino]propanoic acid were determined. The results are shown in Table 12.

[Table 11]

| Examples | Name of Enzyme | Origin of Enzyme | Method for Preparing or Obtaining Enzyme |
|---|---|---|---|
| 35 | Wild Strain Esterase Originated from *Chromobacterium* SC-YM-1 strain | *Chromobacterium chocolatum* | Prepared According to Method Described in Example 7 of JP-3875283. |
| 36 | Esterase Originated from *Chromobacterium* SC-YM-1 strain, 160A189Y363term | *Chromobacterium chocolatum* | Prepared According to Method Described in Example 12 of JP-3486942. |

(continued)

| Examples | Name of Enzyme | Origin of Enzyme | Method for Preparing or Obtaining Enzyme |
|---|---|---|---|
| 37 | Esterase Originated from *Chromobacterium* SC-YM-1 strain, 160S189F363term | *Chromobacterium chocolatum* | Prepared According to Method Described in Example 12 of JP-3486942. |
| 38 | Esterase Originated from *Chromobacterium* SC-YM-1 strain, 160A189F363term | *Chromobacterium chocolatum* | Prepared According to Method Described in Example 12 of JP-3486942. |
| 39 | Esterase Originated from *Chromobacterium* SC-YM-1 strain, V325I | *Chromobacterium chocolatum* | Prepared According to Method Described in Example 2 of JP 2000-78988A. |
| 40 | Esterase Originated from *Chromobacterium* SC-YM-1 strain, T240AV288A | *Chromobacterium chocolatum* | Prepared According to Method Described in Example 2 of JP 2000-78988A. |
| 41 | Esterase Originated from *Chromobacterium* SC-YM-1 strain, N43SA363term | *Chromobacterium chocolatum* | Prepared According to Method Described in Example 2 of JP 2000-78988A. |

[Table 12]

| Examples | Amount of Enzyme (mg) | Conversion Rate (%) | Enantiomeric Excess (%ee) |
|---|---|---|---|
| 35 | 200.6 | 9.2 | 99.1 |
| 36 | 201.7 | 35.2 | 99.8 |
| 37 | 202.7 | 6.9 | 98.9 |
| 38 | 201.4 | 10.1 | 100.0 |
| 39 | 201.3 | 7.8 | 98.9 |
| 40 | 200.2 | 6.8 | 99.0 |
| 41 | 200.8 | 12.0 | 100.0 |

Examples 42 and 43

Production of optically-active 2-benzyl-3-[(N-benzyloxy-N-formyl)amino]propanoic acid

Each of *E. coli* recombinant soluble fractions producing various enzymes shown in Table 13 was weighed into a vessel in the amount shown in Table 14, and 5 mL of a 0.1 M potassium phosphate buffer solution (pH 7.0) and 40.0 mg of an optical isomer mixture (racemate) of 2-benzyl-3-[(N-benzyloxy-N-formyl)amino] propanoic acid ethyl ester were added. This solution was stirred at 25°C for 48 hours, and then 1 mL of an aqueous 3.4% phosphoric acid solution and 10 mL of tert-butyl methyl ether were added, and then mixed. The mixture was allowed to stand still, and then the tert-butyl methyl ether layer was analyzed in the same manner as in Example 1, and the conversion and enantiomeric excess of the resulting optically-active
2-benzyl-3-[(N-benzyloxy-N-formyl) amino]propanoic acid were determined. The results are shown in Table 14.

[Table 13]

| Examples | Name of Enzyme | Origin of Enzyme | Method for Preparing or Obtaining Enzyme |
|---|---|---|---|
| 42 | Esterase Originated from *Chromobacterium* SC-YM-1 strain, 160A189Y363term | *Chromobacterium chocolatum* | Prepared According to Method Described in Example 12 of JP-3486942. |

(continued)

| Examples | Name of Enzyme | Origin of Enzyme | Method for Preparing or Obtaining Enzyme |
|---|---|---|---|
| 43 | Esterase Originated from *Chromobacterium* SC-YM-1 strain, N43SA363term | *Chromobacterium chocolatum* | Prepared According to Method Described in Example 2 of JP 2000-78988A. |

[Table 14]

| Examples | Amount of Enzyme (mg) | Conversion Rate (%) | Enantiomeric Excess (%ee) |
|---|---|---|---|
| 42 | 201.2 | 42.9 | 92.0 |
| 43 | 201.2 | 33.6 | 92.3 |

Conversion Rate (%) = Amount of Product / (Amount of Substrate + Amount of Product) × 100

Enantiomeric Excess (% ee) = (A − B) / (A + B) × 100 (A and B each represent the amount of the corresponding enantiomer, and A > B).

INDUSTRIAL APPLICABILITY

The production method of the present invention is useful for the production of a raw material or an intermediate compound for producing an active pharmaceutical ingredient compound.

## Claims

**1.** A method for producing an optically-active $\alpha$-substituted-$\beta$-amino acid represented by formula (2):

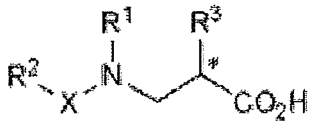

(2)

(wherein $R^1$ represents a hydrogen atom, a $C_1$ to $C_{10}$ acyl group, a carboxyl group or a carboxamide group; $R^2$ represents a hydrogen atom, an optionally-substituted $C_1$ to $C_{10}$ alkyl group, an optionally-substituted $C_6$ to $C_{20}$ aryl group, an optionally-substituted $C_7$ to $C_{20}$ aralkyl group, or a $C_2$ to $C_{20}$ acyl group, an optionally-substituted $C_2$ to $C_{15}$ alkoxycarbonyl group, an optionally-substituted $C_7$ to $C_{20}$ aryloxycarbonyl group, an optionally-substituted $C_8$ to $C_{20}$ aralkyloxycarbonyl group, or an optionally-substituted $C_4$ to $C_{10}$ cyclic ether group; $R^3$ represents an optionally-substituted $C_1$ to $C_{20}$ hydrocarbon group; X represents an oxygen atom or a sulfur atom; and * represents the asymmetric center),
the method comprising a step of asymmetrically hydrolyzing an optical isomer mixture of an α-substituted-β-amino acid ester represented by formula (1):

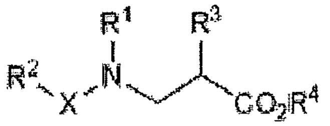

(1)

(wherein $R^4$ represents an optionally-substituted $C_1$ to $C_{10}$ alkyl group; and $R^1$, $R^2$, $R^3$ and X have the same meanings as described above),
using an enzyme capable of asymmetrically hydrolyzing the $-CO_2R^4$ group of the optical isomer mixture or a culture of a microorganism capable of producing the enzyme or a treated product thereof.

**2.** The method according to claim 1, wherein $R^3$ is an optionally-substituted $C_1$ to $C_{10}$ hydrocarbon group.

**3.** The method according to claim 1, wherein $R^3$ is a cyclopentylmethyl group.

**4.** The method according to claim 1, wherein $R^1$ is a $C_1$ to $C_{10}$ acyl group.

**5.** The method according to claim 1, wherein $R^1$ is a formyl group, an acetyl group, a propionyl group or a benzoyl group.

**6.** The method according to claim 1, wherein $R^1$ is a hydrogen group.

**7.** The method according to claim 1, wherein $R^2$ is a $C_7$ to $C_{20}$ aralkyl group.

**8.** The method according to claim 1, wherein $R^2$ is a benzyl group.

**9.** The method according claim 1, wherein $R^4$ is a methyl group or an ethyl group.

**10.** The method according to any one of claim 1 to 9, wherein the enzyme is a hydrolase originated from *Chromobacterium spp.* or *Mucor spp.* microorganism or a variant-type enzyme obtained by deletion, addition or substitution of 1 or 2 of amino acids constituting the hydrolase.

**11.** The method according to any one of claim 1 to 9, wherein the enzyme is a hydrolase originated from *Chromobacterium chocolatum* or *Mucor miehei* microorganism or a variant-type enzyme obtained by deletion, addition or substitution of 1 or 2 of amino acids constituting the hydrolase.

**12.** The method according to any one of claim 1 to 9, wherein the enzyme is an esterase or lipase derived from *Chromobacterium* SC-YM-1 strain (Agency of Industrial Science and Technology: National Institute of Bioscience and Human Technology, accession number: FERM BP-6703) or a variant-type enzyme obtained by deletion, addition or substitution of 1 or 2 of amino acids constituting the esterase or lipase.

**INTERNATIONAL SEARCH REPORT**

International application No. PCT/JP2012/082274

A. CLASSIFICATION OF SUBJECT MATTER
*C12P41/00*(2006.01)i, *C12P13/04*(2006.01)i, *C12N9/18*(2006.01)n

According to International Patent Classification (IPC) or to both national classification and IPC

B. FIELDS SEARCHED

Minimum documentation searched (classification system followed by classification symbols)
C12P41/00, C12P13/04, C12N9/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGESTRY/BIOSIS/MEDLINE(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | QIU,L. et al, Enantioselective hydrogenation of α-aminomethylacrylates containing a free N-H group for the synthesis of β-amino acid derivatives, Proceedings of the National Academy of Sciences of USA., 2007, Vol.104, No.43, p.16787-16792 | 1-12 |
| A | JP 2003-325195 A (Degussa AG.), 18 November 2003 (18.11.2003), claim 9 & US 2004/0029236 A1 & US 2005/0142646 A1 & EP 1361279 A1 & DE 10220739 A & DE 10320211 A & CN 1456675 A | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 January, 2013 (29.01.13) | 05 February, 2013 (05.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/082274

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-5682 A (Sumitomo Chemical Co., Ltd.), 15 January 2009 (15.01.2009), claims 9, 10 & US 2010/0240107 A1 & EP 2154253 A1 & WO 2008/140127 A1 | 10-12 |
| A | JP 7-51090 A (Chisso Corp.), 28 February 1995 (28.02.1995), claims 1, 2 & US 5518903 A | 1-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0061134 A
- JP 3875283 B
- JP 2000078988 A
- JP 3486942 B
- JP 200078988R B

### Non-patent literature cited in the description

- *ARKICOV,* 2010, 196-205
- Molecular Cloning. Cold Spring Harbor Laboratory, 1989
- **OLFERT LANDT et al.** *Gene,* 1990, vol. 96, 125-128
- **SMITH et al.** Genetic Engineering. A Plenum, vol. 31
- **VLASUK ; INOUYE, M. et al.** Experimental Manipulation of Gene Expression. Academic Press
- **HOS.N.HUNT et al.** *Gene,* 1989, vol. 77, 51